# EUROPEAN PATENT APPLICATION

(11) **EP 4 797 278 A1**
(43) Date of publication of application: **26.08.2026**
(21) Application number: 25305226.0
(22) Date of filing: 19.02.2025
(51) Int. Cl.: G16H 40/63, G16H 50/20, G16H 50/30, G16H 50/70

(54) **ECHOCARDIOGRAM CARDIAC MONITORING**

(71) Applicant: Koninklijke Philips N.V., 5656 AE Eindhoven (NL)
(72) Inventor: SHRESHTHA, Kumar, 5656 Eindhoven (NL); CHOFFIN, Benoît, 5656 Eindhoven (NL); PEZZOTTI, Nicola, 5656 Eindhoven (NL)
(74) Representative: Philips Intellectual Property & Standards

(57) **Abstract**

Proposed concepts aim to provide schemes, solutions, concepts, designs, methods, and systems pertaining to cardiac monitoring of a subject which involves obtaining both echocardiogram data and a cardiac activity signal. In particular, embodiments aim to provide a method of cardiac monitoring in which a cardiac rhythm abnormality value is determined and used to generate supplementary data which can accompany the echocardiogram data to provide a comprehensive view of the cardiac behaviour of the subject during a monitoring time period.

## Description

### FIELD OF THE INVENTION

The invention relates to the field of cardiac monitoring, and more specifically to the field of echocardiogram monitoring of cardiac systems.

### BACKGROUND OF THE INVENTION

Echocardiogram exams are routinely carried out to monitor the function and structure of a subject's cardiac system. These examinations may take several minutes and typically involve a sonographer using an ultrasound machine to obtain several echocardiogram images, each displaying different views of the heart at different points in the cardiac cycle. The results of an echocardiogram exam may be used for the detection of heart failure and the diagnosis and monitoring of valvular diseases and congenital heart conditions. Different types of echocardiogram examination may be used for different diagnostic purposes. Types of examination procedure include transthoracic echocardiograms, transoesophageal echocardiograms, stress echocardiograms, and contrast echocardiograms.

Echocardiogram data obtained during an examination can be analysed manually or automatically to derive information about the heart's structure and its movement during the cardiac cycle. Such analysis may be particularly useful for the detection of structural anomalies such as regional wall motion abnormalities. Echocardiogram data does not however provide any information about cardiac electrical activity or rhythm and as such, subject disorders such as heartbeat irregularities and conduction disorders cannot be detected in conventional echocardiogram exams. Any report on the patient that is made by the sonographer at the end of an echo exam may therefore lack crucial information needed for a full assessment of the patient's cardiac health.

### SUMMARY OF THE INVENTION

The invention is defined by the claims.

According to examples in accordance with an aspect of the invention, there is provided a method for monitoring the cardiac system of a subject.

The method comprises: obtaining echocardiogram data and a cardiac activity signal, each describing the behaviour of the subject's cardiac system in a monitoring time period; processing the cardiac activity signal with a machine learning model trained to take a cardiac activity signal as an input and output a prediction of whether the cardiac activity signal describes a cardiac rhythm abnormality event, to determine a cardiac rhythm abnormality value describing the occurrence of cardiac rhythm abnormality events of the subject in the monitoring time period; and generating supplementary data for accompanying the echocardiogram data based on the determined cardiac rhythm abnormality value.

Proposed concepts thus aim to provide schemes, solutions, concepts, designs, methods, and systems pertaining to cardiac monitoring of a subject involving obtaining both echocardiogram data and a cardiac activity signal. In particular, embodiments aim to provide a method of cardiac monitoring in which a cardiac rhythm abnormality value is determined and used to generate supplementary data which can accompany the echocardiogram data to provide a comprehensive view of the cardiac behaviour of the subject during a monitoring time period.

It is not possible to obtain information about cardiac rhythm abnormalities from echocardiogram data, since this data primarily comprises information about the structure of the heart and the surrounding blood vessels. However, echocardiogram examinations typically last several minutes and therefore provide an ideal window for detecting sporadic or intermittent rhythm abnormalities which cannot be detected over short monitoring windows. This may be particularly true for rhythm abnormalities in their nascent state, when episodes are often short and spaced out, such as in paroxysmal atrial fibrillation.

In the proposed invention, both a cardiac activity signal and echocardiogram data describing the behaviour of the subject's cardiac system in a monitoring time period are obtained. A trained machine learning model is then used to analyse the cardiac activity signal to determine a cardiac rhythm abnormality value comprising information about any cardiac rhythm abnormality events that may have occurred in the monitoring time period. This value can then be used to generate supplementary data for accompanying the echocardiogram data. The invention therefore allows for the detection and documentation of abnormal rhythm episodes that occur during an echocardiogram, ensuring the echo examination provides a comprehensive view of the cardiac health the subject.

Sonographers performing echocardiogram examinations may not be trained to obtain and interpret other forms of cardiac data and therefore may not be able to accurately identify cardiac rhythm abnormality events from a cardiac activity signal. The present invention is advantageous in that interpretation of the cardiac activity signal is done automatically by the machine learning model, removing any requirement that the sonographer can interpret the data themselves. It has been shown that the use of machine learning models may indeed be more accurate than technicians in analysing cardiac activity signals and therefore the proposed invention may provide for accurate and reliable cardiac rhythm abnormality detection and analysis.

Hence, the proposed invention leverages both echocardiogram data and a cardiac activity signal to obtain a comprehensive view of the behaviour of the subject's cardiac system during the monitoring time period. The cardiac activity signal is automatically processed using a pre-trained machine learning model to generate a cardiac rhythm abnormality value which may accurately describe the occurrence of cardiac rhythm abnormality events in the monitoring time period.

Ultimately, an improved method of echocardiogram monitoring of a subject may be provided by the proposed concepts(s).

In some embodiments, the method may further comprise generating a cardiac report describing the cardiac activity of the subject in the monitoring time period, and the cardiac report may comprise one or more frames of the echocardiogram data and the supplementary data. This may advantageously combine the echocardiogram results and the cardiac rhythm abnormality findings into a digestible report that can be presented to a cardiologist to improve assessment and diagnosis of the subject.

In some embodiments, the method may further comprise selecting the one or more frames of the echocardiogram data for the cardiac report based on the cardiac activity signal, and optionally wherein selecting the one or more frames comprises determining, based on the cardiac activity signal, the time at which a specific point in the cardiac cycle of the subject occurs. Cardiac activity signals obtained alongside echocardiogram data may also be used in a gating mechanism to select appropriate frames of the obtained echocardiogram data for use in subject monitoring. Using the cardiac data for both echocardiogram gating and cardiac rhythm abnormality analysis may improve the efficiency of the cardiac monitoring system by reducing the number of monitoring devices required.

In some embodiments, the echocardiogram data may describe the behaviour of the cardiac system of the subject in a first time period in the monitoring time period, and the cardiac activity signal may describe the behaviour of the cardiac system of the subject in a second time period in the monitoring time period, wherein the first and second time period at least partially overlap. Thus, the echocardiogram data and the cardiac activity signal may provide at least partially concurrent cardiac monitoring, advantageously allowing for structural events seen in the echocardiogram data to be mapped directly to cardiac activity events described by the cardiac activity signal.

In some embodiments, the first and second time period may be substantially the same. This advantageously allows for the echocardiogram data and the cardiac activity signal to be obtained simultaneously in the same examination, saving patient and practitioner time.

In some embodiments, the cardiac rhythm abnormality value may comprise at least one of: a number of cardiac rhythm abnormality events; a type of cardiac rhythm abnormality event; a duration of a cardiac rhythm abnormality episode; a frequency of cardiac rhythm abnormality events; a type of heartbeat; a measurement of a cardiac axis; a measurement of a cardiac interval; and a signal amplitude measurement. Each of these values may provide valuable information about the subject's cardiac rhythm that can be used alongside the echocardiogram data to provide a more complete and accurate picture of the cardiac behaviour of the subject in the monitoring time period.

In some embodiments, the cardiac rhythm abnormality value may comprise a plurality of cardiac rhythm abnormality sub-values and processing the cardiac activity signal with the machine learning model may comprise segmenting the cardiac activity signal into a plurality of cardiac activity signal segments, and processing each of the plurality of cardiac activity signal segments with the machine learning model to determine a respective plurality of cardiac rhythm abnormality sub-values. This may improve the time resolution of the cardiac rhythm abnormality analysis.

In some embodiments, the cardiac rhythm abnormality value may comprise a plurality of cardiac rhythm abnormality sub-values, and generating the supplementary data may comprise generating a summary statistic value describing the plurality of cardiac rhythm abnormality sub-values. This may facilitate easier and more effective patient care via the production of digestible and easily interpretable data for use by a clinical health practitioner in assessing the cardiac health of the subject.

In some embodiments, the cardiac activity signal may comprise at least one of:
a signal obtained from a wearable smart device; a PPG signal; a blood pressure signal; and an ECG signal, and optionally wherein the ECG comprises at least one of: a 2-lead ECG signal; a 3-lead ECG signal; a 5-lead ECG signal; a 12-lead ECG signal; a 16-lead ECG signal; and a Holter ECG signal. Each of these signals may advantageously provide a cardiac activity signal from which information about the occurrence of cardiac rhythm abnormality events can be derived and used to supplement the echocardiogram data.

In some embodiments the cardiac activity signal may comprise an ECG signal and the machine learning model may be trained using a deep learning algorithm configured to receive an array of training inputs and respective known outputs, and a training input of the machine learning model may comprise a Holter ECG signal and a respective known output may comprise a known cardiac rhythm abnormality value associated with the Holter ECG signal. Using Holter ECG data to train the machine learning model may be advantageous since due to the variations in the placement of different Holter recorders, models trained on such data may be lead agnostic. Therefore, the resulting cardiac rhythm abnormality may be more accurate.

In some embodiments, obtaining the echocardiogram data and the cardiac activity signal may comprise performing a transthoracic echocardiogram (TTE). This type of examination typically lasts several minutes and therefore allows sufficient time for sporadic or intermittent cardiac rhythm abnormalities to be detected.

Another aspect of the present invention comprises a computer program comprising computer program code means adapted, when said computer program is run on a computer, to implement any of the methods for cardiac monitoring outlined above.

A third aspect of the present invention comprises a cardiac monitoring system. The system comprises: a data acquisition module configured to obtain echocardiogram data and a cardiac activity signal, each describing the behaviour of the subject's cardiac system in a monitoring time period; and a processing arrangement configured to: process the cardiac activity signal with a machine learning model trained to take a cardiac activity signal as an input and output a prediction of whether the cardiac activity signal describes a cardiac rhythm abnormality event, to determine a cardiac rhythm abnormality value describing the occurrence of cardiac rhythm abnormality events of the subject in the monitoring time period; and generate supplementary data for accompanying the echocardiogram data based on the determined cardiac rhythm abnormality value.

This cardiac monitoring system may be combined with monitoring sensors in a subject monitoring system. The present invention therefore further considers a subject monitoring system comprising: an echocardiogram sensor configured to acquire echocardiogram data describing the behaviour of the subject's cardiac system in a monitoring time period; a cardiac activity sensor configured to acquire a cardiac activity signal describing the behaviour of the subject's cardiac system in the monitoring time period; and the cardiac monitoring system described above. In some embodiments the cardiac activity sensor may comprise an ECG sensor.

Embodiments may be employed in combination with conventional/existing patient monitoring methods and systems. In this way, embodiments may integrate into legacy systems to improve and/or extend their functionality and capabilities. An improved echocardiogram monitoring system that automatically generates supplementary data based on a generated cardiac rhythm abnormality value may therefore be provided by proposed embodiments.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for cardiac monitoring of a subject according to a proposed embodiment;
Fig. 2 is a simplified flow diagram of a work flow for automated cardiac rhythm abnormality detection during an echocardiography exam according to another proposed embodiment;
Fig. 3 is a simplified block diagram of a subject monitoring system according to another aspect of the invention; and
Fig. 4 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems, and methods, are intended for the purpose of illustration only and are not intended to limit the scope of the invention. These and other features, aspects, and advantages of the apparatus, systems, and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Implementations in accordance with the present disclosure relate to various techniques, methods, schemes, and/or solutions pertaining to cardiac monitoring of a subject. According to proposed concepts, a number of possible solutions may be implemented separately or jointly. That is, although these possible solutions may be described below separately, two or more of these possible solutions may be implemented in one combination or another.

Embodiments of the proposed invention aim to provide a method for combined echocardiogram examination and cardiac rhythm abnormality monitoring. A cardiac activity signal is obtained and then automatically analysed using a machine learning model to determine a cardiac rhythm abnormality value. This value is then used to generate supplementary data for accompanying the echocardiogram data. This may advantageously result in more complete cardiac monitoring of the subject without requiring the presence of a health care professional that is trained to interpret and detect cardiac rhythm abnormalities.

By way of summary, the proposed methods and systems involve the following concepts:
A. Data Acquisition: echocardiogram data and a cardiac activity signal are obtained that each describe the behaviour of the subject's cardiac system in a monitoring time period. The echocardiogram data and the cardiac activity signal may in some instances be taken simultaneously during the same patient exam which may be a transthoracic echocardiogram, transoesophageal echocardiogram, stress echocardiogram, or a contrast echocardiogram. The cardiac activity signal may be a PPG signal, a blood pressure signal and/or an ECG signal, all of which describe cardiac activity information.
B. Cardiac Rhythm Abnormality Analysis: a machine learning model is trained to output a cardiac rhythm abnormality value describing the occurrence of cardiac rhythm abnormality events in a certain monitoring time period. The cardiac rhythm abnormality value may comprise one or more quantitative or qualitative values that describe whether any cardiac rhythm abnormality events have occurred during the monitoring time period and/or a characteristic of a cardiac rhythm abnormality event that has occurred in the monitoring time period.
C. Suplementary Data Generation: based on the determined cardiac rhythm abnormality value, supplementary data is generated that can be used to accompany the echocardiogram data to provide additional insights to medical practitioners involved in diagnosis and monitoring of the subject's cardiac health. Generating the supplementary data may involve aggregation and further processing steps being performed on the output from the machine learning model.

Embodiments of the present invention therefore employ a machine-learning algorithm to perform cardiac rhythm abnormality analysis. A machine-learning algorithm is any self-training algorithm that processes input data in order to produce or predict output data. Here, the input data would be the cardiac activity signal and the output data would be the cardiac rhythm abnormality value describing the occurrence of cardiac rhythm abnormality events of a subject in a monitoring time period.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives. Purely by way of example, the machine learning model may employ a Convolutional Neural Network.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g., the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

There are several types of neural network, such as convolutional neural networks (CNNs) and recurrent neural networks (RNNs). CNNs typically contain several layers, including a convolutional layer, a pooling layer, and a fully connected layer. The convolutional layer consists of a set of learnable filters and extracts features from the input. The pooling layer is a form of non-linear down-sampling, reducing the data size by combining the outputs of a plurality of neurons in one layer into a single neuron in the next layer. The fully connected layer connects each neuron in one layer to all the neurons in the next layer.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, weightings of the mathematical operation of each neuron may be modified until the error converges. Known methods of modifying a neural network include gradient descent, backpropagation algorithms and so on.

In the context of the present invention the training input data entries for the machine learning model may be real or simulated cardiac activity signals describing the cardiac activity of a real or simulated patient respectively. The training output data entries correspond to precalculated cardiac rhythm abnormality values. In other words, the machine learning model may be trained using a deep learning algorithm configured to receive an array of training inputs and respective known outputs, wherein a training input comprises a cardiac activity signal and a respective known output comprises a known cardiac rhythm abnormality value associated with the input cardiac activity signal. The known cardiac rhythm abnormality values may have been manually obtained from the training input data by trained health care professionals. In this way, the machine learning model may be trained to automatically process a cardiac activity signal to predict whether the cardiac activity signal describes a cardiac rhythm abnormality event.

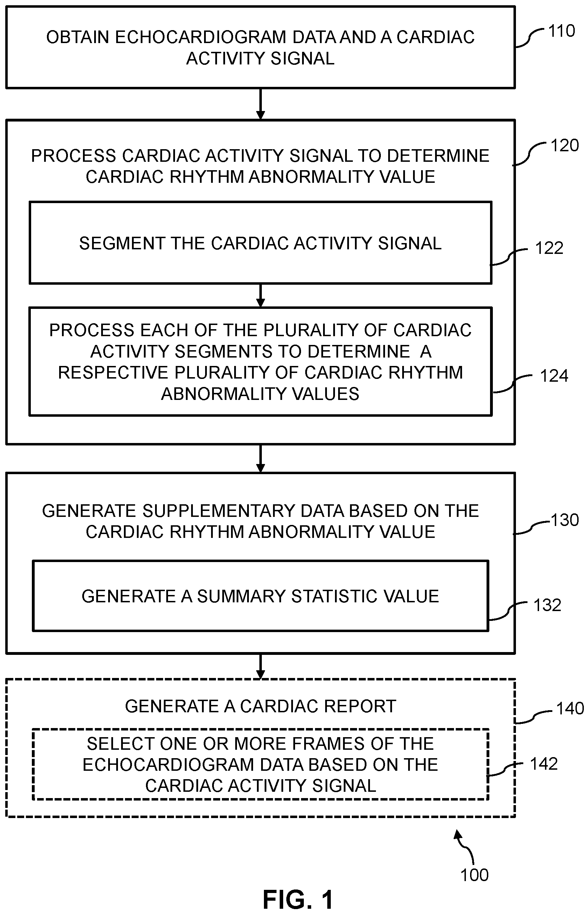

Referring now to Fig. 1, there is depicted a simplified flow diagram of a method 100 for cardiac monitoring of a subject according to a proposed embodiment.

The method commences with a step 110 comprising obtaining echocardiogram data and a cardiac activity signal, each describing the behaviour of the subject's cardiac system in a monitoring time period. In this particular embodiment, the cardiac activity signal is a 3-lead ECG signal, and both the cardiac activity signal and the echocardiogram data are obtained during a transthoracic echocardiogram (TTE) that is performed on the patient. 3-lead ECG data may already be being obtained during a TTE for use in echocardiogram gating and so this type of echocardiogram exam is a prime candidate for the proposed concepts of the present invention.

In this exemplary embodiment, the echocardiogram data describes the behaviour of the cardiac system of the subject in a first time period in the monitoring time period, and the cardiac activity signal describes the behaviour of the cardiac system of the subject in a second time period in the monitoring time period, wherein the first and second time period are substantially the same. Therefore, in this embodiment, concurrent echocardiogram and cardiac activity monitoring of the subject are carried out during a transthoracic echocardiogram to simultaneously acquire both echocardiogram data and a cardiac activity signal which both describe the behaviour of subject's cardiac system during a common monitoring time period.

Once the echocardiogram data and the cardiac activity signal have been obtained, the method proceeds to step 120 comprising processing the cardiac activity signal with a machine learning model trained to take a cardiac activity signal as an input and output a prediction of whether the cardiac activity signal describes a cardiac rhythm abnormality event, to determine a cardiac rhythm abnormality value describing the occurrence of cardiac rhythm abnormality events of the subject in the monitoring time period.

In this embodiment of the invention, in which the cardiac activity signal comprises an ECG signal, the machine learning model is trained using a deep learning algorithm configured to receive an array of training inputs and respective known outputs, wherein a training input of the machine learning model is a Holter ECG signal and a respective known output is a cardiac rhythm abnormality value associated with the Holter ECG signal.

12-lead ECG signals are often used for ECG assessments since they provide a comprehensive view of the heart from multiple directions in the longitudinal and transverse planes. There is therefore a large availability of 12-lead ECG patient data and information about the cardiac rhythm abnormalities the data describe that could be used to train the machine learning model of the present invention. However, other forms of ECG signal may be more suitable for use alongside echocardiogram examination. For example, 3-lead ECG may be preferred as it easier to set up and is more suitable for use in ECG gating as described below. The electrode placement in a 3-lead ECG set up is very different from that in a 12-lead system. Therefore, although ground truth 12-lead ECG data with annotated cardiac rhythm abnormality values may be easily available, machine learning models trained on such data may have difficulty generalizing to a 3-lead system. Since 3-lead ECG data is not often analysed by trained clinical professionals to assess cardiac anomalies, annotated ground truth 3-lead data may be difficult or impossible to obtain in sufficient quantities for training the machine learning model.

A possible solution to this problem is to use a deep learning algorithm for training the machine learning model that can analyse rhythm abnormalities in a lead agnostic manner. In other words, no matter the position of the ECG electrodes, the model learns to find the features of the ECG relevant for the diagnosis and detection of cardiac rhythm abnormality events, without focussing on the morphology of the specific waveforms which can vary from one lead position to another. Holter recorders are small, wearable ECG devices that may be used over the course of one or more days to detect irregular cardiac activity. Due to the variations in the placement of Holter records during use, models trained on a large amount of Holter data coming from different devices have the property of being lead agnostic. This makes them suitable for training the machine learning model used in the method 100.

In this example embodiment of the invention, processing the cardiac activity signal with the machine learning model further comprises sub-steps 122 and 124. Step 122 comprises segmenting the cardiac activity signal into a plurality of cardiac activity signal segments. For example, a continuous 3-lead ECG signal may be split into 30 second segments. However, the invention is not limited to this segmentation and both shorter and longer segment lengths are envisioned. For example, each of the plurality of cardiac activity signal segments may describe the cardiac activity of the subject over a respective plurality of segment time periods, wherein each of the plurality of segment time periods has a duration in the range 1-60 seconds.

Step 124 comprises processing each of the plurality of cardiac activity signal segments with the machine learning model to determine a respective plurality of cardiac rhythm abnormality sub-values. The cardiac activity value determined in step 120 may therefore comprise a plurality of cardiac rhythm abnormality sub-values that each describe the occurrence of cardiac rhythm abnormality events of the subject in a different segment of the cardiac activity signal. This may improve the temporal resolution of any cardiac rhythm abnormality findings. In this example each of the cardiac rhythm abnormality sub-values comprises a categorisation of the type of each detected cardiac rhythm abnormality event.

There are many different ways in which a subject can display cardiac rhythm abnormality. These may be categorised into different types of cardiac rhythm abnormality event. For example, a cardiac rhythm abnormality event may comprise at least one of: an abnormal QRS interval, bradycardia, tachycardia, a sinus rhythm, a premature supraventricular contraction (PSVC), a ventricular ectopic beat (VEB), a PSVC couplet, a VEB couplet, ventricular trigeminy, atrial fibrillation or flutter, supraventricular tachycardia, ventricular run, a second degree atrioventricular bock, a third degree atrioventricular block, and a ventricular pause.

The method then proceeds to a step 130 comprising generating supplementary data for accompanying the echocardiogram data based on the determined cardiac rhythm abnormality value. In this embodiment, generating the supplementary data comprises a step 132 comprising generating a summary statistic value describing the plurality of cardiac rhythm abnormality sub-values. For example, the summary statistic value may comprise the total number of each type of cardiac rhythm abnormality event predicted by the machine learning model to have occurred during the monitoring time period.

The method 100 further comprises optional step 140, which comprises generating a cardiac report describing the cardiac activity of the subject in the monitoring time period, wherein the cardiac report comprises one or more frames of the echocardiogram data and the supplementary data. Thus, at least a portion of the echocardiogram data and the generated supplementary data is compiled to generate a cardiac report which may be used by a clinical health professional to assess the cardiac health of the subject. In this example, step 140 comprises a sub-step 142 which involves selecting the one or more frames of the echocardiogram data for the cardiac report based on the cardiac activity signal. This may involve determining, based on the cardiac activity signal, the time at which a specific point in the cardiac cycle of the subject occurs. This may be known as a gating process, where analysis of cardiac activity data is used to trigger the acquisition and/or selection of echocardiogram frames to ensure high quality echocardiogram data is obtained. For example, concurrent cardiac activity data may be used to select echocardiogram frames corresponding to particular points in the subject's cardiac cycle.

The method 100 may therefore provide an improved method for performing a transthoracic echocardiogram with improved efficiency and resulting in more detailed insights into the cardiac health of a subject. TTE exams may typically last several minutes. A cardiac activity signal (for example a 3-lead ECG signal) may be obtained throughout the exam to assist with image acquisition through a process known as gating in which the cardiac activity signal is used to select frames of the echocardiogram data for different cardiac cycles to minimise imaging artifacts and improve temporal resolution. However, this gating process only requires a small amount of data around the echocardiogram frames and therefore using the cardiac activity signal only for gating may result in large portions of the collected signal being unused. The echocardiogram data gathered in the exam can be used for analysing cardiac structure and function but is not suitable for use in heart rhythm analysis. The method 100 may therefore advantageously use a cardiac activity signal to enhance the findings of an echocardiogram with cardiac rhythm abnormality information that may be needed for the diagnosis of conduction disorders and atrial fibrillation. Sonographers performing cardiac echocardiography are not usually skilled in performing rhythm interpretation. The proposed method is therefore particularly advantageous in that the rhythm abnormality analysis is performed in a completely automated way without requiring the input of a clinical professional trained to analyse and diagnose cardiac rhythm abnormality disorders.

The method 100 described above is just one possible embodiment of the proposed invention. Although step 110 of the method 100 in this example comprises performing a transthoracic echocardiogram, this need not be the case in other embodiments. The concepts and features of the proposed invention may be equally well applied to other types of echocardiogram examination such as transoesophageal echocardiograms, stress echocardiograms, and contrast echocardiograms.

Further, although in this example the echocardiogram data and the cardiac activity signal describe the behaviour of the subject's cardiac system over the same time period, this need not be the case in other embodiments. For example, in another embodiment the echocardiogram data describes the behaviour of the cardiac system of the subject in a first time period in the monitoring time period, and the cardiac activity signal describes the behaviour of the cardiac system of the subject in a second time period in the monitoring time period, wherein the first and second time period only partially overlap. In other embodiments, the first and second time period may not overlap at all. Thus, the echocardiogram data and the cardiac activity signal may each describe a portion of the monitoring time period and may be obtained sequentially or concurrently during the monitoring time period.

In this particular embodiment, the cardiac activity signal comprises a 3-lead ECG signal. However, the invention is not limited to such and in other embodiments of the proposed invention the cardiac activity signal may comprise at least one of: a signal obtained from a wearable smart device; a PPG signal; a blood pressure signal; and an ECG signal. Any of these signals may be suitable for analysis of cardiac rhythm abnormality events and optionally for use in a gating process for selecting ECG frames. If the cardiac data is an ECG signal it may comprise at least one of: a 2-lead ECG signal; a 3-lead ECG signal; a 5-lead ECG signal; a 12-lead ECG signal; a 16-lead ECG signal; and a Holter ECG signal.

Similarly, other aspects of the method 100 may be implemented differently in alternative embodiments. The machine learning model used in step 120 of the method 100, need not be trained on Holter ECG training data. Suitable training data for training the machine learning model may be selected based on the form of the cardiac activity signal and the availability of large amounts of real or simulated patient data. For example, the machine learning model may be trained on at least one of: a signal obtained from a wearable smart device; a PPG signal; a blood pressure signal; a 2-lead ECG signal; a 3-lead ECG signal; a 5-lead ECG signal; a 12-lead ECG signal; and a 16-lead ECG signal. Further, step 120 need not comprise the sub-steps 122 and 124. The machine learning model may be trained to process the cardiac activity signal as a whole rather than segments of the signal.

The cardiac rhythm abnormality value may be any value providing information on the occurrence of cardiac rhythm abnormality events of the subject in the monitoring time period. In particular, the cardiac rhythm abnormality value may comprise at least one of: a number of rhythm abnormalities; a type of cardiac rhythm abnormality; a duration of a cardiac rhythm abnormality episode; a frequency of cardiac rhythm abnormality events; a type of heart beat; a measurement of a cardiac axis; a measurement of a cardiac interval; and a signal amplitude measurement. Thus, the cardiac rhythm abnormality value may describe detected cardiac rhythm abnormality events or may describe features of the cardiac activity signal that may indicate such events. For example, the machine learning model may be trained to extract from a cardiac activity signal comprising an ECG signal a measurement of the P axis, the QRS axis and/or the T axis, a measurement of a P, PR, and/or QRS intervals and/or a measurement of the ST elevation. Each of these values may indicate the presence of certain cardiac rhythm abnormalities when they lie within a certain range.

The generation of supplementary data in step 130 need not comprise generating a summary statistic value. In some implementations of the present invention, the supplementary data may simply comprise the cardiac rhythm abnormality value, and therefore no further processing is required to be carried out. In other embodiments, inputs from a clinical health care professional and/or other known physiological and demographic parameters of the patient may be used alongside the cardiac rhythm abnormality value in order to generate the supplementary data for accompanying the echocardiogram data.

Step 140 of the method 100, involving the generation of the cardiac report, is an optional step in the proposed invention. In other embodiments, the supplementary data may simply be stored in the subject's medical records. Additionally, or alternatively, the supplementary data may be displayed alongside the echocardiogram data to the health care professional carrying out the echocardiogram exam via a display device. It is further not necessary that the cardiac activity data be used for an echocardiogram gating process. A separate form of data may be obtained for this function, or gating may not be performed at all.

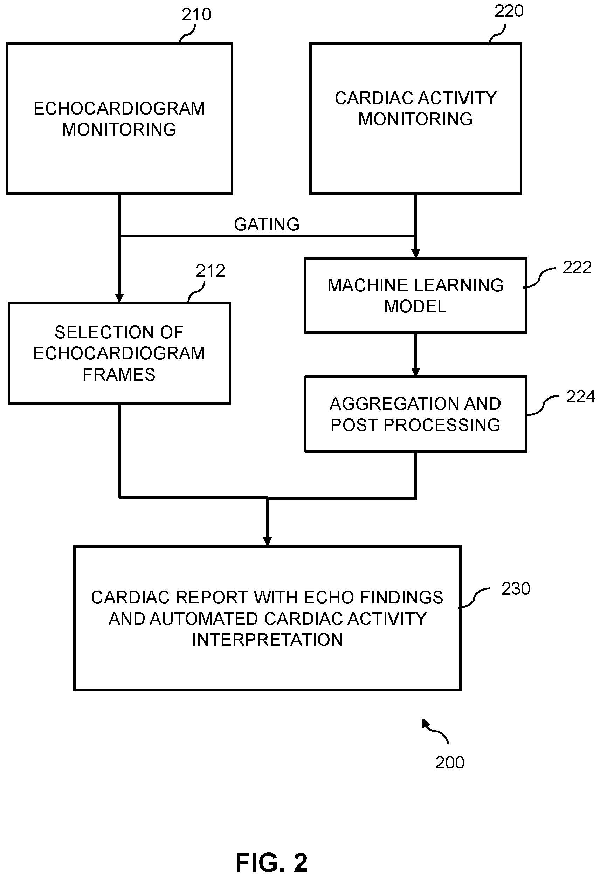

Referring now to Fig. 2 there is depicted a is a simplified flow diagram of a workflow 200 for automated cardiac rhythm abnormality detection during an echocardiography exam according to a proposed embodiment of the invention.

In the workflow 200, echocardiogram monitoring 210 and cardiac activity monitoring 220 are both performed to obtain echocardiogram data and a cardiac activity signal respectively. The cardiac activity signal may be used in a gating process, as described above, to aid in the selection in step 212 of one or more echocardiogram frames to be used in analysis of the structure and function of the subject's cardiac system.

In a step 222 the cardiac activity signal is processed with a machine learning model which returns a cardiac rhythm abnormality value describing abnormalities detected in the cardiac activity signal. The machine learning model comprises a cardiac activity signal interpretation algorithm which provides as output a summary of the events encountered during exam, the key measurements performed on the cardiac activity signal as well as any episodes of cardiac rhythm abnormalities encountered in the exam. The results of the machine learning model are then postprocessed and aggregated in step 224 into report ready metrics describing the type, occurrence, and frequency of detected cardiac rhythm abnormality events. This may be referred to as supplementary data for accompanying the echocardiogram data.

In addition to the rhythm abnormality analysis, other values may be derived from the cardiac activity signal to enhance findings from the echocardiogram. For example, the cardiac activity signal may be processed to detect the present of regional wall motion abnormalities that may facilitate the assessment of ischemia. Other structural cardiac abnormalities that can be detected from echocardiogram data may also benefit from being paired with a plethysmograph or electrical view of the heart.

The selected echocardiogram frames and the generated supplementary data are then summarised in a cardiac report in a step 230. The cardiac report describes the cardiac behaviour and cardiac health of the subject and may be used by a cardiologist in assessment of the subject.

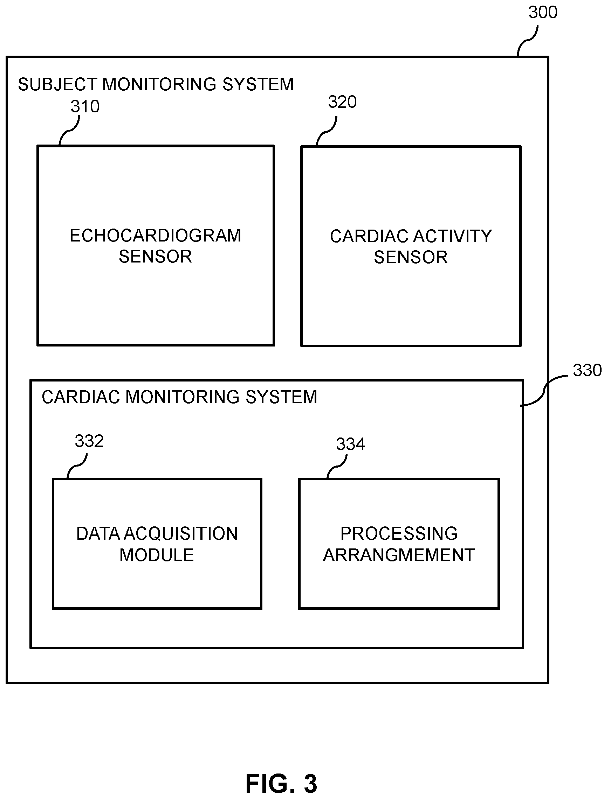

Referring now to Fig. 3, there is depicted a simplified block diagram of a patient monitoring system 300 according to another aspect of the proposed invention.

The patient monitoring system 300 comprises an echocardiogram sensor 310 and a cardiac activity sensor 320. The echocardiogram sensor 310 is configured to acquire echocardiogram data describing the behaviour of the subject's cardiac system in a monitoring time period, and the cardiac activity sensor 320 is configured to acquire a cardiac activity signal describing the behaviour of the subject's cardiac system in the monitoring time period. Any conventional or novel form of echocardiogram sensor and cardiac activity sensor may be used. For example, the cardiac activity sensor may comprise at least one of: a wearable smart device; a PPG sensor; a blood pressure sensor; and an ECG sensor.

The patient monitoring system 300 further comprises a cardiac monitoring system 330. The cardiac monitoring system comprises a data acquisition module 332 configured to obtain the echocardiogram data and cardiac activity signal from the echocardiogram sensor and the cardiac activity sensor respectively. The cardiac monitoring system 330 further comprises a processing arrangement 334 configured to: process the cardiac activity signal with a machine learning model trained to take a cardiac activity signal as an input and output a prediction of whether the cardiac activity signal describes a cardiac rhythm abnormality event, to determine a cardiac rhythm abnormality value describing the occurrence of cardiac rhythm abnormality events of the subject in the monitoring time period; and generate supplementary data for accompanying the echocardiogram data based on the determined cardiac rhythm abnormality value.

Thus, the proposed concepts of the present invention may be implemented in a subject monitoring system 300. In some embodiments, the subject monitoring system may comprise further components such as additional monitoring sensors, and input and output interfaces for obtaining and displaying patient information.

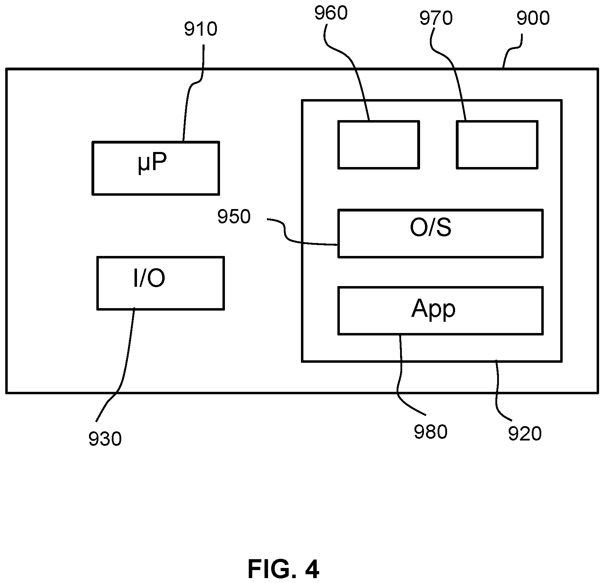

Fig. 4 illustrates an example of a computer 900 within which one or more parts of an embodiment may be employed. Various operations discussed above may utilize the capabilities of the computer. For example, one or more parts of a proposed embodiment may be incorporated in any element, module, application, and/or component discussed herein. In this regard, it is to be understood that system functional blocks can run on a single computer or may be distributed over several computers and locations (e.g., connected via internet), such as a cloud-based computing infrastructure.

The computer 900 includes, but is not limited to, PCs, workstations, laptops, PDAs, palm devices, servers, storages, and the like. Generally, in terms of hardware architecture, the computer 900 may include one or more processors 910, memory 920, and one or more I/O devices 930 that are communicatively coupled via a local interface (not shown). The local interface can be, for example but not limited to, one or more buses or other wired or wireless connections, as is known in the art. The local interface may have additional elements such as controllers, buffers (caches), drivers, repeaters, and receivers, to enable communications. Further, the local interface may include address, control, and/or data connections to enable appropriate communications among the aforementioned components.

The processor 910 is a hardware device for executing software that can be stored in the memory 920. The processor 910 can be virtually any custom made or commercially available processor, a central processing unit (CPU), a digital signal processor (DSP), or an auxiliary processor among several processors associated with the computer 900, and the processor 910 may be a semiconductor based microprocessor (in the form of a microchip) or a microprocessor.

The memory 920 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 920 may incorporate electronic, magnetic, optical, and/or other types of storage media. Note that the memory 920 can have a distributed architecture, where various components are situated remote from one another, but can be accessed by the processor 910.

The software in the memory 920 may include one or more separate programs each of which comprises an ordered listing of executable instructions for implementing logical functions. The software in the memory 920 includes a suitable operating system (O/S) 950, compiler 960, source code 970, and one or more applications 980 in accordance with exemplary embodiments. As illustrated the application 980 comprises numerous functional components for implementing the features and operations of the exemplary embodiments. The application 980 of the computer 900 may represent various applications, computational units, logic, functional units, processes, operations, virtual entities, and/or modules in accordance with exemplary embodiments, but the application 980 is not meant to be a limitation.

The operating system 950 controls the execution of other computer programs, and provides scheduling, input-output control, file and data management, memory management, and communication control and related services. It is contemplated by the inventors that the application 980 for implementing exemplary embodiments may be applicable on all commercially available operating systems.

Application 980 may be a source program, executable program (object code), script, or any other entity comprising a set of instructions to be performed. When a source program, then the program is usually translated via a compiler (such as the compiler 960), assembler, interpreter, or the like, which may or may not be included within the memory 920, so as to operate properly in connection with the O/S 950. Furthermore, the application 980 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 930 may include input devices such as, for example but not limited to, a mouse, keyboard, scanner, microphone, camera, etc. Furthermore, the I/O devices 930 may also include output devices, for example but not limited to a printer, display, etc. Finally, the I/O devices 930 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 900 is a PC, workstation, intelligent device or the like, the software in the memory 920 may further include a basic input output system (BIOS) (omitted for simplicity). The BIOS is a set of essential software routines that initialize and test hardware at start-up, start the O/S 950, and support the transfer of data among the hardware devices. The BIOS is stored in some type of read-only-memory, such as ROM, PROM, EPROM, EEPROM or the like, so that the BIOS can be executed when the computer 900 is activated.

When the computer 900 is in operation, the processor 910 is configured to execute software within the memory 920, to communicate data to and from the memory 920, and to generally control operations of the computer 900 pursuant to the software. The application 980 and the O/S 950 are read, in whole or in part, by the processor 910, perhaps buffered within the processor 910, and then executed.

When the application 980 is implemented in software it should be noted that the application 980 can be stored on virtually any computer readable medium for use by or in connection with any computer related system or method. In the context of this document, a computer readable medium may be an electronic, magnetic, optical or other physical device or means that can contain or store a computer program for use by or in connection with a computer related system or method.

The application 980 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed method(s) and system(s) may be implemented in hardware or software, or a mixture of both (for example, as firmware running on a hardware device). To the extent that an embodiment is implemented partly or wholly in software, the functional steps illustrated in the process flow diagrams may be performed by suitably programmed physical computing devices, such as one or more central processing units (CPUs) or graphics processing units (GPUs). Each process - and its individual component steps as illustrated in the flow diagrams - may be performed by the same or different computing devices. According to embodiments, a computer-readable storage medium stores a computer program code configured to cause one or more physical computing devices to carry out a quality assessment method as described above when the program is run one the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that one or more programs stored thereon can be loaded into a processor.

To the extent that an embodiment is implemented partly or wholly in hardware, some of the blocks shown in the block diagrams may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPAGs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flow diagrams and block diagrams in the Figures illustrate the architecture, functionality, and operation of possible implementations of systems, methods, and computer program products according to various embodiments of the present invention. **In** this regard, each block in the flow diagrams or block diagrams may represent a module segment, or portion of instruction, which comprises one or more executable instructions for implementing the specified logical function(s). **In** some alternative implementations, the functions noted in the block may occur out of the order noted in the figures. For example, two blocks shown in succession may, in face be executed substantially concurrently, or the blocks may sometimes be executed in reverse order, depending on the functionality involved. It will also be noted that each block of the block diagrams and/or flow diagrams and combinations of blocks in the block diagrams and/or flow diagrams, can be implemented by special purpose hardware-based systems that perform the specified functions or acts or carry out combinations of special purpose hardware and computer instructions.

## Claims

1. A method (100) for monitoring the cardiac system of a subject, the method comprising:
obtaining (110) echocardiogram data and a cardiac activity signal, each describing the behaviour of the subject's cardiac system in a monitoring time period;
processing (120) the cardiac activity signal with a machine learning model trained to take a cardiac activity signal as an input and output a prediction of whether the cardiac activity signal describes a cardiac rhythm abnormality event, to determine a cardiac rhythm abnormality value describing the occurrence of cardiac rhythm abnormality events of the subject in the monitoring time period; and
generating (130) supplementary data for accompanying the echocardiogram data based on the determined cardiac rhythm abnormality value.

2. The method of claim 1, wherein the method further comprises generating (140) a cardiac report describing the cardiac activity of the subject in the monitoring time period, and
wherein the cardiac report comprises one or more frames of the echocardiogram data and the supplementary data.

3. The method of claim 2, wherein the method further comprises:
selecting (142) the one or more frames of the echocardiogram data for the cardiac report based on the cardiac activity signal, and optionally wherein selecting the one or more frames comprises determining, based on the cardiac activity signal, the time at which a specific point in the cardiac cycle of the subject occurs.

4. The method of any preceding claim, wherein the echocardiogram data describes the behaviour of the cardiac system of the subject in a first time period in the monitoring time period, and the cardiac activity signal describes the behaviour of the cardiac system of the subject in a second time period in the monitoring time period, and
wherein the first and second time period at least partially overlap.

5. The method of claim 4, wherein the first and second time period are substantially the same.

6. The method of any preceding claim, wherein the cardiac rhythm abnormality value comprises at least one of:
a number of rhythm abnormality events;
a type of cardiac rhythm abnormality;
a duration of a cardiac rhythm abnormality episode;
a frequency of cardiac rhythm abnormality events;
a type of heart beat;
a measurement of a cardiac axis;
a measurement of a cardiac interval; and
a signal amplitude measurement.

7. The method of any preceding claim, wherein the cardiac rhythm abnormality value comprises a plurality of cardiac rhythm abnormality sub-values and wherein processing the cardiac activity signal with the machine learning model comprises:
segmenting (122) the cardiac activity signal into a plurality of cardiac activity signal segments; and
processing (124) each of the plurality of cardiac activity signal segments with the machine learning model to determine a respective plurality of cardiac rhythm abnormality sub-values.

8. The method of any preceding claim wherein the cardiac rhythm abnormality value comprises a plurality of cardiac rhythm abnormality sub-values, and
wherein generating the supplementary data comprises generating (132) a summary statistic value describing the plurality of cardiac rhythm abnormality sub-values.

9. The method of any preceding claim wherein the cardiac activity signal comprises at least one of:
a signal obtained from a wearable smart device;
a PPG signal;
a blood pressure signal; and
an ECG signal,
and optionally wherein the ECG signal comprises at least one of:
a 2-lead ECG signal;
a 3-lead ECG signal;
a 5-lead ECG signal;
a 12-lead ECG signal;
a 16-lead ECG signal; and
a Holter ECG signal.

10. The method of any preceding claim, wherein the cardiac activity signal comprises an ECG signal and the machine learning model is trained using a deep learning algorithm configured to receive an array of training inputs and respective known outputs, and
wherein a training input of the machine learning model is a Holter ECG signal and a respective known output is a known cardiac rhythm abnormality value associated with the Holter ECG signal.

11. The method of any preceding claim, wherein obtaining the echocardiogram data and the cardiac activity signal comprises performing a transthoracic echocardiogram (TTE).

12. A computer program comprising computer program code means adapted, when said computer program is run on a computer, to implement the method of any of claims 1-11.

13. A cardiac monitoring system (330) comprising:
a data acquisition module (332) configured to obtain echocardiogram data and a cardiac activity signal, each describing the behaviour of the subject's cardiac system in a monitoring time period; and
a processing arrangement (334) configured to:
process the cardiac activity signal with a machine learning model trained to take a cardiac activity signal as an input and output a prediction of whether the cardiac activity signal describes a cardiac rhythm abnormality event, to determine a cardiac rhythm abnormality value describing the occurrence of cardiac rhythm abnormality events of the subject in the monitoring time period; and
generate supplementary data for accompanying the echocardiogram data based on the determined cardiac rhythm abnormality value.

14. A subject monitoring system (300) comprising:
an echocardiogram sensor (310) configured to acquire echocardiogram data describing the behaviour of the subject's cardiac system in a monitoring time period;
a cardiac activity sensor (320) configured to acquire a cardiac activity signal describing the behaviour of the subject's cardiac system in the monitoring time period; and
the cardiac monitoring system (330) of claim 13.

15. The subject monitoring system of claim 14, wherein the cardiac activity sensor comprises an ECG sensor.
